(19) Europäisches Patentamt
European Patent Office
Office européen des brevets

(11) EP 3 601 218 B1

(12) # FASCICULE DE BREVET EUROPEEN

(45) Date de publication et mention de la délivrance du brevet:
**05.05.2021 Bulletin 2021/18**

(21) Numéro de dépôt: **18714328.4**

(22) Date de dépôt: **19.03.2018**

(51) Int Cl.:
***C07C 303/02*** (2006.01) ***C07C 309/15*** (2006.01)

(86) Numéro de dépôt international:
**PCT/FR2018/050653**

(87) Numéro de publication internationale:
**WO 2018/172677 (27.09.2018 Gazette 2018/39)**

(54) **NOUVEAU PROCEDE D'OBTENTION DE L'ACIDE 2-ACRYLAMIDO-2-METHYLPROPANE SULFONIQUE**

NEUES VERFAHREN ZUR HERSTELLUNG VON 2-ACRYLAMID-2-METHYLPROPANSULFONSÄURE

NEW METHOD FOR PRODUCING 2-ACRYLAMIDO-2-METHYLPROPANE SULPHONIC ACID

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **20.03.2017 FR 1752289**

(43) Date de publication de la demande:
**05.02.2020 Bulletin 2020/06**

(73) Titulaire: **SPCM SA**
**42160 Andrézieux-Bouthéon (FR)**

(72) Inventeurs:
- **FAVERO, Cédrick**
**42160 Andrezieux Boutheon (FR)**
- **KIEFFER, Johann**
**42160 Andrezieux Boutheon (FR)**

(74) Mandataire: **Cabinet Laurent & Charras**
**Le Contemporain**
**50 Chemin de la Bruyère**
**69574 Dardilly Cedex (FR)**

(56) Documents cités:
**CN-A- 103 664 709**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

## Description

### DOMAINE DE L'INVENTION

Le domaine de l'invention concerne un nouveau procédé d'obtention de l'acide 2-acrylamido-2-méthylpropane sulfonique. Plus précisément la présente invention a pour objet un procédé de fabrication de l'acide 2-acrylamido-2-méthylpropane sulfonique consistant à faire réagir entre eux l'acrylonitrile, l'acide sulfurique fumant et l'isobutylène.

### ETAT ANTERIEUR DE LA TECHNIQUE

L'acide 2-acrylamido-2-méthylpropane sulfonique est largement utilisé comme additif dans les fibres acryliques, ou encore comme matière première pour obtenir des polymères utilisés en tant que dispersant, hydrogel ou épaississant dans divers secteurs comme l'industrie pétrolière, la construction, le traitement des eaux (dessalement de l'eau de mer, industrie minérale, etc...) ou la cosmétique.

La réaction mise en œuvre dans le procédé de préparation de l'acide 2-acrylamido-2-méthylpropane sulfonique répond au schéma réactionnel ci-dessous, dans lequel l'acrylonitrile est présent en excès de manière à être à la fois le solvant de la réaction et un réactif. L'acrylonitrile est mis en contact avec de l'acide sulfurique fumant (oléum) et de l'isobutylène.

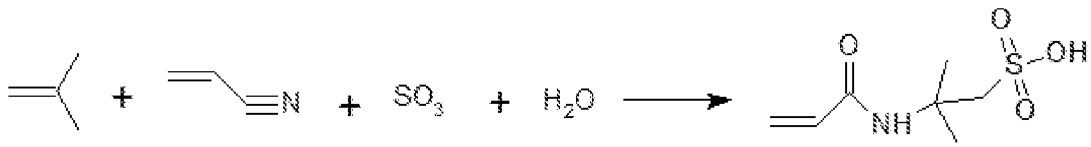

L'acide 2-acrylamido-2-méthylpropane sulfonique n'est pas soluble dans le solvant acrylonitrile, en conséquence, le produit de réaction est sous une forme de suspension de cristaux dans le solvant de réaction. En revanche, l'acide 2-acrylamido-2-méthylpropane sulfonique est soluble dans l'eau.

A titre d'exemples, les documents US 6,448,347 et CN 102351744 décrivent un procédé de fabrication de l'acide 2-acrylamido-2-méthylpropane sulfonique selon un mode continu.

L'acide 2-acrylamido-2-méthylpropane sulfonique est par la suite séparé de l'acrylonitrile, généralement par filtration et peut être purifié ultérieurement par plusieurs méthodes connues. En effet, une purification est nécessaire car un faible taux d'impuretés présent dans l'acide 2-acrylamido-2-méthylpropane sulfonique affecte fortement sa polymérisation, et plus particulièrement le poids moléculaire et le taux d'insolubles dans l'eau des polymères et copolymères en résultant.

Ainsi, dans le document WO 2009/072480 qui porte sur un procédé de fabrication d'acide 2-acrylamido-2-méthylpropanesulfonique (ATBS) il est expliqué que les impuretés de type acide 2-méthyl-2-propényl-1-sulfonique (IBSA) et acide 2-méthylidène-1,3-propylènedisulfonique (IBDSA) affectent fortement la polymérisation au-delà d'une certaine concentration.

Le document US 4,337,215 décrit une méthode de purification de l'acide 2-acrylamido-2-méthylpropane sulfonique par recristallisation dans de l'acide acétique, par dissolution à chaud et cristallisation par rampe de refroidissement. Malgré la bonne pureté de l'acide 2-acrylamido-2-méthylpropane sulfonique obtenu, le procédé dont le rendement est limité, fait intervenir de multiples étapes de dissolution/refroidissement et requiert un traitement de l'acide acétique usagé pour le régénérer par distillation avant réutilisation ultérieure dans un nouveau batch de recristallisation de l'acide 2-acrylamido-2-méthylpropane sulfonique.

Le séchage de l'acide 2-acrylamido-2-méthylpropane sulfonique est nécessaire afin de diminuer la quantité d'acrylonitrile et d'acrylamide restant présent dans le cristal. Ces 2 composés ont une classification comme cancérogène, mutagène ou toxique pour la reproduction (CMR), il est donc nécessaire de procéder à une filtration efficace pour essorer au mieux l'acrylonitrile, et ensuite de sécher afin d'obtenir des teneurs en acrylonitrile et acrylamide qui soient faibles.

Le document CN 103664709 décrit un procédé de préparation d'acide 2-acrylamido-2-méthylpropane sulfonique permettant de s'affranchir de cette étape de séchage longue et coûteuse. L'étape de séchage est substituée par une étape de lavage à l'acide acétique glacial dans lequel l'ATBS n'est pas soluble. Bien que ce procédé permette de raccourcir la durée de la synthèse d'acide 2-acrylamido-2-méthylpropane sulfonique, la consommation de solvant et l'énergie thermique nécessaire à la purification par recristallisation sont encore importants.

Comme il est décrit dans l'article intitulé « On the Ritter synthesis of N-tert-butylacrylamide, reaction between tert-butylalcohol and acrylonitrile in non-aqueous system » publié dans « Iranian J. of Polymer Science and Technology Vol 4 No 1, p.42-49, 1995 », auteur Demetra Dragan, le rendement en acide 2-acrylamido-2-méthylpropane sulfonique est lié au ratio de $SO_3$ libre dans le milieu de synthèse. Sans être lié à une quelconque théorie, plus le milieu est sulfonant,

plus la réaction est sélective envers l'acide 2-acrylamido-2-méthylpropane sulfonique au détriment du N-tert-butylacrylamide.

Il existe de nombreuses méthodes permettant d'obtenir l'acide 2-acrylamido-2-méthylpropane sulfonique. Toutefois, il semble accepté qu'il est important de pouvoir améliorer le procédé d'obtention en réduisant la quantité de solvant utilisé et en supprimant l'étape de séchage, ou en diminuant la quantité d'énergie thermique utilisée, tout en gardant une bonne qualité d'acide 2-acrylamido-2-méthylpropane sulfonique au regard de son taux d'impuretés, de ses capacités à polymériser sous forme de polymères de haut poids moléculaires ou encore au taux de composés cancérogène, mutagène ou toxique pour la reproduction

## PRESENTATION DE L'INVENTION

La présente invention a pour objet un procédé de fabrication de l'acide 2-acrylamido-2-méthylpropane sulfonique comprenant au moins les étapes successives suivantes :

1) mélange d'acrylonitrile avec au moins un composé apporteur de $SO_3$ à une température comprise entre -80 et 30°C pendant au moins 1 seconde afin d'obtenir un mélange sulfonant,
2) mise en contact et mélange d'isobutylène et du mélange sulfonant avec un ratio molaire $SO_3$:isobutylène compris entre 0,2:1 et 2:1 et un ratio molaire acrylonitrile:isobutylène compris entre 3:1 et 60:1, à une température comprise entre -40 et 100°C pendant un temps compris entre 10 secondes et 300 minutes afin d'obtenir un mélange réactionnel,
3) séparation solide/liquide du mélange réactionnel et isolement des particules solides contenues dans le mélange réactionnel sous la forme d'une composition 1 dans laquelle les particules solides représentent de 50 à 99% en poids de la composition 1,
4) mélange de la composition 1 avec une solution aqueuse A pendant au moins 10 minutes à une température comprise entre -20 et 70°C afin d'obtenir une suspension 1 de cristaux d'acide 2-acrylamido-2-méthylpropane sulfonique,
5) séparation solide/liquide de la suspension 1 et isolement des cristaux sous la forme d'une composition 2 dans laquelle les cristaux représentent entre 40 et 99 % en poids de la composition 2.

Par « étapes successives », on désigne des étapes qui se suivent chronologiquement. En d'autres termes, des étapes successives sont réalisées dans l'ordre indiqué et ne peuvent pas être interverties. En revanche, une ou plusieurs étapes intermédiaires peuvent, éventuellement, être intercalées entre deux étapes successives.

Dans la description de l'invention, les plages de valeurs incluent les bornes. Par exemple, la plage de valeurs « entre -40°C et 80°C » inclut les valeurs -40°C et 80°C. D'autre part, la description divulgue toutes les combinaisons possibles entre les bornes des différentes plages de valeurs. Par exemple, la divulgation des plages -80 à 30°C, avantageusement -40 à 10°C inclut notamment les plages -80°C à 10°C, 10°C à 30°C, -80°C à -40°C ou -40°C à 30°C.

<u>Etape 1</u>

Le composé apporteur de $SO_3$ est généralement de l'acide sulfurique fumant, utilisé à une concentration comprise entre 100% et 113,5%. A titre d'exemple, l'acide sulfurique fumant à 113,5% comprend 60% en poids de $SO_3$.

Selon un autre mode de réalisation, le composé apporteur de $SO_3$ et de l'eau peuvent également être ajoutés séparément. Les formes alpha, beta ou gamma du $SO_3$ peuvent être utilisées indifféremment dans la présente invention. L'eau peut également provenir d'acide sulfurique ayant une concentration inférieure à 100% en $H_2SO_4$.

Dans un autre mode de réalisation particulier, le $SO_3$ peut être mélangé avec de l'acide sulfurique.

Dans le cadre de la présente invention, l'acrylonitrile peut être utilisé sous sa forme anhydre ou sous forme de solution aqueuse contribuant ainsi à la balance en eau de cette étape.

Dans le cas où l'acrylonitrile est utilisé sous forme aqueuse la réaction entre l'eau contenue dans l'acrylonitrile et le composé de $SO_3$ produit un acide sulfurique ayant une concentration équivalente de 96 à 103%.

$$\%H_2SO_4\ \text{effectif} = \frac{m_{SO_3} * \%H_2SO_{4_{SO_3}} + m_{oleum} * \%H_2SO_{4_{oleum}} + m_{H_2SO_4} * \%H_2SO_4}{\frac{m_{ACN} * \%MC}{100} + m_{SO_3} + m_{oleum} + m_{H_2SO_4} + \sum_i \frac{m_i^{solvant\ i} * \%MC_i}{100}}$$

% $H_2SO_4$ effectif : représente le taux d'acide sulfurique résultant du mélange entre l'acrylonitrile avec au moins un composé apporteur de $SO_3$

$m_{oleum}$: masse d'oléum

$\%H_2SO_{4_{oleum}}$ : concentration de l'oléum, exprimé en $\%H_2SO_4$.
$m_{SO_3}$ : masse $SO_3$
$\%H_2SO_{4_{SO_3}}$ : concentration du $SO_3$, exprimée en $\%H_2SO_4$.
$m_{H_2SO_4}$ : masse d'acide sulfurique
$\%H_2SO_4$ : concentration de l'acide sulfurique, exprimé en $\%H_2SO_4$
$m_{ACN}$: masse d'acrylonitrile
%MC : pourcentage d'eau en poids contenu dans l'acrylonitrile
$m_i^{solvant\ i}$ = masse du solvant i
$\%MC_i$= pourcentage d'eau en poids contenu dans le solvant i

Usuellement, la concentration de l'oléum n'est pas exprimée en $\%H_2SO_4$ mais en pourcentage de $SO_3$ libre. Auquel cas, la formule ci-dessous s'applique pour convertir en poids du pourcentage de $SO_3$ en pourcentage d'$H_2SO_4$.

$$\%H_2SO_4 = 100 + \frac{(\%SO_3) * M_{H_2O}}{M_{SO_3}}$$

$\%H_2SO_4$ : concentration de l'acide sulfurique, exprimé en $\%H_2SO_4$

$M_{H_2O}$ : masse molaire de l'eau

$M_{SO_3}$ : masse molaire du $SO_3$

$\%SO_3$ : pourcentage poids du $SO_3$ libre dans l'oléum.

Dans le cas particulier où le $SO_3$ est utilisé sous forme gazeuse (pur ou dilué dans un gaz vecteur), le $\%SO_3$ est de 100% donc le $\%H_2SO_4$ correspondant est de 122,5%.
Dans un mode de réalisation particulier, l'étape 1) comprend le mélange d'acrylonitrile avec au moins un composé apporteur de $SO_3$ dans un solvant 1.
De manière non limitative, le solvant 1 est choisi parmi l'anhydride acétique, les acides carboxyliques tel que l'acide acétique, les nitriles, les alcools, les amines, les alcanes, les amides, les éthers, les aromatiques, les acides alkylsulfoniques et la phase liquide résultant de la séparation liquide/solide de l'étape 3). D'une manière préférée l'étape 1) contient en tant que solvant 1 uniquement de l'acrylonitrile.
Pendant cette étape, la température de mélange est comprise entre -80 et 30°C, de préférence comprise entre -80 et 20°C, préférentiellement comprise entre -40 et 10°C.
Le temps de mélange est avantageusement compris entre 1 seconde et 600 minutes, préférentiellement entre 5 secondes et 120 minutes.
Le mélange des réactifs de l'étape 1) peut s'effectuer par diverses technologies. A titre d'exemples et de manière non limitative, nous pouvons citer les réacteurs avec agitateur, les réacteurs boucle, les mélangeurs statiques, les microréacteurs, les réacteurs piston.

Etape 2

Pendant cette étape, l'isobutylène peut être introduit dans le mélange sulfonant sous forme gazeuse, pur ou dilué avec un gaz neutre (par exemple l'azote ou l'argon), ou bien sous forme de gaz liquéfié, ou dissous dans un solvant 2. Préférentiellement l'isobutylène est introduit dissous dans un solvant 2. Préférentiellement, ce solvant 2 est l'acrylonitrile ou la phase liquide résultant de la séparation liquide/solide de l'étape 3).
La réaction d'introduction de l'isobutylène peut être faite sous pression atmosphérique ou bien sous pression plus élevée, par exemple jusqu'à 50 bars relatifs.
L'isobutylène peut être fabriqué selon différentes méthodes connues de l'homme de métier. A titre d'exemple et de manière non limitative, l'isobutylène peut être obtenu par la déshydratation du tert-butanol ou de l'isobutanol, par la déshydrogénation de l'isobutane, par l'isomérisation du but-1-ène ou du but-2-ène, par la fermentation du glucose ou de déchets de dérivés ligno cellulosiques à l'aide de microorganismes ou par le craquage du méthyl-tertiobutyléther (MTBE).
Le ratio molaire $SO_3$:isobutylène est compris entre 0,2:1 et 2:1, préférentiellement entre 0,4:1 et 1,5:1, plus préférentiellement entre 0,7:1 et 1,2:1.

Le ratio molaire entre le composé de $SO_3$ et l'isobutylène est définit de la façon suivante :

$$SO_3 : IB = \frac{\left[\frac{m_{SO_3} * \%H_2SO_{4_{SO_3}} + m_{oleum} * \%H_2SO_{4_{oleum}} + m_{H_2SO_4} * \%H_2SO_4}{M_{H_2SO_4}}\right] / 100}{\frac{m_{IB}}{M_{IB}}}$$

$m_{IB}$ : masse d'isobutylène
$M_{IB}$ : masse molaire d'isobutylène
$SO_3$ : nombre de moles de $SO_3$
IB : nombre de moles d'isobutylène
$M_{H_2SO_4}$ : masse molaire de $H_2SO_4$

Les autres paramètres $m_{oleum}$, $\%H_2SO_{4_{oleum}}$, $m_{H_2SO_4}$, $\%H_2SO_4$, $m_{SO_3}$, $\%H_2SO_{4_{SO_3}}$ sont les mêmes que ceux décrits précédemment.

Le ratio molaire acrylonitrile:isobutylène est compris entre 3:1 et 60:1, préférentiellement entre 4:1 et 40:1, plus préférentiellement entre 6:1 et 20:1.

La température est comprise entre -40 et 100°C, de préférence entre -40 et 80°C, plus préférentiellement comprise entre -20 et 70°C.

Le temps de mélange entre l'isobutylène et le mélange sulfonant de l'étape 1) est compris entre 10 secondes et 300 minutes, préférentiellement entre 1 minute et 120 minutes.

Le mélange des réactifs de l'étape 2) peut s'effectuer par diverses technologies. A titre d'exemples et de manière non limitative, nous pouvons citer les réacteurs avec agitateurs, les réacteurs boucles, les mélangeurs statiques, les microréacteurs, les réacteurs pistons.

Lors de l'introduction de l'isobutylène en étape 2), les particules solides de l'acide 2-acrylamido-2-méthylpropane sulfonique se forment et précipitent car elles ne sont pas solubles dans le mélange sulfonant issu de l'étape 1). En conséquence, le produit de réaction est sous la forme d'une suspension de particules solides dans le mélange réactionnel.

Le mélange réactionnel, à l'issue de l'étape 2), a un taux de solide en poids avantageusement compris entre 5 et 40%, plus préférentiellement entre 10 et 35% et encore plus préférentiellement entre 15 et 30%.

Le taux de solide est définit selon l'expression suivante :

$$\text{Taux solide (\%)} = 100 * \frac{\frac{m_{IB}}{M_{IB}} * M_{ATBS}}{\sum_i m_i^{\text{solvant i}} + m_{SO_3} + m_{ACN} + m_{IB} + m_{oleum} + m_{H_2O} + m_{H_2SO_4}}$$

$M_{ATBS}$ : masse molaire de l'acide 2-acrylamido-2-méthylpropane sulfonique
$m_{H_2O}$ : masse d'eau

Les autres paramètres $m_i^{\text{solvant i}}$, $m_{SO_3}$, $m_{oleum}$, $m_{H_2SO_4}$, $m_{ACN}$, $m_{IB}$, $M_{IB}$ sont les mêmes que ceux décrits précédemment.

Le mélange réactionnel peut être immédiatement engagé dans l'étape 3) ou bien être stocké temporairement. Le stockage temporaire du mélange réactionnel peut être réalisé à une température avantageusement inférieure à 50°C, plus préférentiellement inférieure à 25°C.

Selon un mode de réalisation particulier, de l'eau peut être ajoutée au mélange réactionnel afin de consommer le $SO_3$ libre qui n'a pas été consommé lors de la réaction. L'eau peut être ajoutée sous forme pure ou bien sous forme de solution aqueuse contenant des sels ou des composés solubles ou miscibles. A titre d'exemple et de manière non limitative, l'eau peut être introduite sous forme de solution comprenant de l'acrylonitrile et/ou de l'acide 2-acrylamido-2-méthylpropane sulfonique et/ou un alcool comprenant de 1 à 4 carbones et/ou un acide inorganique.

Etape 3

Les particules d'acide 2-acrylamido-2-méthylpropane sulfonique obtenus à l'issue de l'étape 2) sont isolées grâce à une séparation liquide/solide. A titre d'exemples et de manière non limitative, nous pouvons citer l'utilisation d'une centrifugeuse verticale ou horizontale, d'un décanteur, d'un filtre presse, d'un filtre à bande, d'un filtre à disques, d'un filtre poussoir, ou d'un filtre à tambour rotatif. La séparation liquide/solide peut également s'effectuer par décantation gravitaire.

Dans un mode de réalisation particulier, le mélange réactionnel peut être concentré en particules d'acide 2-acrylamido-2-méthylpropane sulfonique par évaporation des solvants 1 et 2 avant l'étape de séparation solide/liquide.

Selon un mode particulier de l'invention, la phase liquide résultant de la séparation peut être utilisée en tant que solvant 1 et/ou 2 dans les étapes 1) et 2). Cette phase liquide peut être utilisée avec ou sans purification préalable. Comme techniques de purification de la phase liquide, nous pouvons citer à titre d'exemples, la distillation fractionnée, l'évaporation, la pervaporation, la neutralisation avec une base organique ou inorganique, et l'extraction liquide/liquide.

Préférentiellement après l'étape de séparation liquide/solide, les particules d'acide 2-acrylamido-2-méthylpropane sulfonique ne sont pas séchées.

En pratique, les particules d'acide 2-acrylamido-2-méthylpropanesulfonique du mélange réactionnel obtenu à l'issue de l'étape 2) sont isolées sous la forme d'une composition 1 dans laquelle les particules d'acide 2-acrylamido-2-méthylpropanesulfonique représentent de 50 à 99%, de préférence entre 50 et 97%, plus préférentiellement entre 60 et 95%, plus préférentiellement entre 70 et 90% en poids de la composition 1.

D'une manière générale, dans la description, le taux de particules solides dans la composition 1 représente le ratio entre le poids total de particules d'acide 2-acrylamido-2-méthylpropanesulfonique et le poids total de la composition 1. Le restant de la composition 1 peut être composé d'eau, d'acrylonitrile, d'isobutylène, d'acide sulfurique ou tout autre composé utilisé pendant le procédé, ou impuretés formées durant la synthèse.

Dans cette étape, la composition 1 à base de particules d'acide 2-acrylamido-2-méthylpropanesulfonique peut contenir de l'acrylonitrile.

Selon un mode particulier de l'invention, la composition 1 peut être lavée avec un solvant 3, de préférence une solution d'acrylonitrile (humide ou anhydre). Avantageusement, la quantité de solvant 3 de lavage mis en œuvre varie généralement entre 0,5 et 10 équivalent en poids par rapport à la quantité de particules solides d'acide 2-acrylamido-2-méthylpropanesulfonique isolées.

Etape 4

Le produit de réaction dans la composition 1 en mélange avec la solution aqueuse A est sous la forme d'une suspension, appelée suspension 1. En d'autres termes, dans l'étape 4), une partie du produit de réaction (particules solides) de la composition 1 est solubilisée dans la solution aqueuse A et une partie n'est pas solubilisée, ce qui conduit à la formation de la suspension 1. En pratique, lors du mélange de la composition 1 avec la solution aqueuse A, la phase aqueuse est saturée en produit de réaction (particules solides solubilisées). La totalité du produit de réaction (particules solides) ne peut donc pas se solubiliser. Ainsi, à aucun moment lors de l'étape 4) une solution dépourvue de particules solides n'est observée puisque la phase aqueuse est saturée et des particules solides de la composition 1 restent à l'état solide contrairement à une étape de recristallisation.

Grâce à l'étape 4), moins de solvant et moins d'énergie thermique sont consommés en comparaison d'une étape de recristallisation comme celle décrite dans le document US 4,337,215 auquel se réfère le document CN 103664709. En effet, lors d'une recristallisation, les cristaux sont solubilisés, puis recristallisés. La mise en solution de cristaux permet de dissoudre d'éventuelles impuretés présentes dans les cristaux. La recristallisation permet de ne pas piéger ces impuretés à nouveau dans les cristaux. L'étape 4) selon l'invention n'est pas comparable à une recristallisation étant donné que les particules solides ne sont pas toutes dissoutes simultanément, une suspension 1 étant formée.

Le mélange de la composition 1 avec une solution aqueuse A peut s'effectuer par diverses technologies. A titre d'exemples et de manière non limitative, nous pouvons citer les réacteurs avec agitateurs, les réacteurs boucles, les mélangeurs statiques, les microréacteurs, les réacteurs pistons.

Il n'y a pas de limitation quant à l'ordre d'addition entre la composition 1 et la solution aqueuse A. On peut d'abord introduire la composition 1 puis la solution aqueuse A, ou inversement.

Dans un autre mode de réalisation, la composition 1 et la solution aqueuse A peuvent être introduites simultanément.

Le ratio en poids de solution aqueuse A mélangée avec la composition 1 de l'étape 3) est avantageusement compris entre 0,05:1 et 1:1 (solution aqueuse A/composition 1), plus préférentiellement entre 0,15:1 et 0,9:1.

Avantageusement, la solution aqueuse A peut comprendre jusqu'à 20% en poids en solvant 4 organique, préférentiellement de 0 à 15 % en poids de solvant 4 organique, plus préférentiellement de 2 à 10% en poids de solvant 4 organique.

Selon un mode particulier de l'invention, la solution aqueuse A peut comprendre au moins 80% en poids d'eau et jusqu'à 20% en poids de solvant 4 organique, préférentiellement entre 85% et 100% en poids d'eau et de 0% à 15% en poids de solvant 4 organique, plus préférentiellement entre 90% et 98% en poids d'eau et 2% à 10% en poids de solvant 4 organique.

Le solvant 4 organique est avantageusement choisi parmi les acides carboxyliques comprenant de 1 à 8 carbones, les amides comprenant de 1 à 8 carbones, les alcools comprenant de 1 à 8 carbones, les cétones comprenant de 1 à 8 carbones, les éthers comprenant de 1 à 8 carbones, les esters comprenant de 1 à 8 carbones, les alcanes comprenant de 1 à 8 carbones, les hydrocarbures halogénés comprenant de 1 à 8 carbones, les nitriles comprenant de 1 à 8 carbones ou leurs mélanges. Préférentiellement le solvant 4 est choisi parmi l'acrylonitrile, l'isopropanol, l'acide acétique et leurs mélanges. Préférentiellement le solvant 4 est l'acrylonitrile.

Selon un autre mode particulier de l'invention, la solution aqueuse A peut comprendre au moins 80% en poids d'eau et jusqu'à 20% en poids d'acide inorganique, préférentiellement entre 80% et 99% en poids d'eau et de 1% à 20 % en poids d'acide inorganique, plus préférentiellement entre 85% et 98% en poids d'eau et 2% à 15% en poids d'acide inorganique. Préférentiellement, l'acide inorganique est l'acide sulfurique.

La solution aqueuse A peut également comprendre un solvant 4 organique et un acide inorganique.

Selon un mode particulier, la solution aqueuse A peut comprendre jusqu'à 55% en poids d'acide 2-acrylamido-2-méthylpropane sulfonique.

Selon un mode particulier de l'invention, le mélange en étape 4) peut s'effectuer sous une pression absolue inférieure ou égale à 1 bar. Cette pression permet de séparer tout ou partie du solvant 4 et/ou de l'acide inorganique et/ou de l'eau éventuellement présent dans la suspension 1. Cette pression peut être utilisée durant toute la durée du mélange ou partiellement.

Le temps de mélange entre la solution aqueuse A et la composition 1 est avantageusement compris entre 10 et 720 minutes, plus préférentiellement entre 30 et 600 minutes.

La température pendant la mise en contact et pendant le mélange, entre la solution aqueuse A et la composition 1, est comprise entre -20°C et 70 °C, préférentiellement entre -20°C et 50°C, plus préférentiellement entre 5°C et 50°C et encore plus préférentiellement entre 10°C et 40 °C. Selon un autre mode de réalisation particulier, la température peut être comprise entre 5 °C et 70°C.

La température est donc moins élevée que dans le procédé décrit dans le document CN 103664709. Il est, en effet, important que le produit de réaction dans la composition 1 ne soit pas entièrement solubilisé dans la solution aqueuse A, contrairement à l'étape de recristallisation du document CN 103664709.

Ainsi, lors de l'étape 4), l'homme du métier saura adapter la température en fonction du pourcentage massique en particules solides de la composition 1, et ce, afin d'obtenir une suspension : la suspension 1.

La suspension 1, après le mélange, a un taux de cristaux non solubilisés avantageusement compris entre 10 et 85%, préférentiellement entre 20 et 40% en poids.

Etape 5

Les cristaux d'acide 2-acrylamido-2-méthylpropane sulfonique contenus dans la suspension 1 et obtenus à l'issue de l'étape 4) sont isolés par une étape de séparation liquide/solide et se présentent sous la forme d'une composition 2. A titre d'exemples et de manière non limitative, nous pouvons citer l'utilisation d'une centrifugeuse, d'un décanteur, d'un filtre presse, d'un filtre à bande, d'un filtre à disques, d'un filtre clos sous vide, d'un filtre clos sous pression ou d'un filtre à tambour rotatif. D'une manière préférentielle, la séparation liquide/solide s'effectue à l'aide d'une centrifugeuse ou d'un filtre clos de type Nutshe.

Les cristaux obtenus après cette étape de séparation solide/liquide peuvent être utilisés tel quel ou bien séchés. A titre d'exemples et de manière non limitative, nous pouvons citer l'utilisation de toutes technologies de séchage par convection, conduction ou rayonnement, (Sécheur à lit fluidisé, lit traversé, séchage sur bande convoyeuse, séchage micro-onde, séchage par rayonnement haute fréquence, infra rouge, séchage par atomisation)

L'opération de séchage peut être réalisée à pression atmosphérique ou bien sous vide.

L'opération de séchage peut être réalisée de manière discontinue (en batch) ou continue.

Préférentiellement, après l'étape de séparation liquide/solide, les cristaux ne sont pas séchés.

La composition 2 isolée a un taux de cristaux d'acide 2-acrylamido-2-méthylpropane sulfonique compris entre 40 et 99%, de préférence entre 60 et 99%, plus préférentiellement entre 60 et 98% en poids. Le restant de la composition comprend principalement de l'eau.

D'autre part, la phase liquide obtenue à la suite de la séparation liquide/solide contient principalement de l'eau et de l'acide 2-acrylamido-2-méthylpropane sulfonique à saturation, et minoritairement du solvant organique 1 et/ou 2 et/ou 3 et/ou 4 ou de l'acide inorganique. Selon un mode particulier de l'invention, cette phase liquide après séparation peut être utilisée totalement ou partiellement dans la solution aqueuse A en étape 4).

Etape 6

Dans une étape 6) optionnelle, la composition 2 contenant les cristaux obtenus à l'issue de l'étape 5) est lavée à l'aide d'une solution de lavage.

La solution de lavage est une solution aqueuse qui peut comprendre jusqu'à 20% en poids de solvant 4 organique.

Préférentiellement, la solution de lavage comprend au moins 80% en poids d'eau et jusqu'à 20% en poids de solvant 4 organique, plus préférentiellement entre 80% et 99% en poids d'eau et de 1% à 20 % en poids de solvant 4 organique, et encore plus préférentiellement entre 85% et 98% en poids d'eau et 2% à 15% en poids de solvant 4 organique.

Comme déjà dit, le solvant 4 organique est avantageusement choisi parmi les acides comprenant de 1 à 8 carbones, les amides comprenant de 1 à 8 carbones, les alcools comprenant de 1 à 8 carbones, les cétones comprenant de 1 à 8 carbones, les éthers comprenant de 1 à 8 carbones, les esters comprenant de 1 à 8 carbones, les alcanes comprenant de 1 à 8 carbones, les hydrocarbonés halogénés comprenant de 1 à 8 carbones, les nitriles comprenant de 1 à 8 carbones ou leurs mélanges. Préférentiellement, le solvant est choisi parmi l'acrylonitrile, l'isopropanol, l'acide acétique ou leurs mélanges, plus préférentiellement le solvant 4 est l'acrylonitrile.

Selon un mode particulier de l'invention, le lavage de la composition 2 obtenue à l'issue de l'étape 5) peut s'effectuer en pulvérisant de la solution de lavage sur ladite composition 2.

Selon un autre mode particulier de l'invention, le lavage de la composition 2 obtenue à l'issue de l'étape 5) peut s'effectuer en mettant en suspension la composition 2 dans la solution de lavage.

Le ratio en poids entre la solution aqueuse de lavage et la composition 2 obtenue à l'issue de l'étape 5) est avantageusement compris entre 0,05:1 et 10:1 (solution aqueuse de lavage/composition 2), et plus préférentiellement entre 0,1:1 et 5:1.

Les cristaux d'acide 2-acrylamido-2-méthylpropane sulfonique obtenus à l'issue de cette sixième étape optionnelle sont avantageusement isolés de la solution de lavage, par exemple, par une étape de séparation liquide/solide, sous la forme d'une composition 3. A titre d'exemples et de manière non limitative, nous pouvons citer l'utilisation d'une centrifugeuse verticale ou horizontale, d'un décanteur, d'un filtre presse, d'un filtre à bande, d'un filtre à disques, d'un filtre poussoir, d'un filtre clos sous vide, d'un filtre clos sous pression, d'un atomiseur, d'un sécheur bicone ou d'un filtre à tambour rotatif. La séparation liquide/solide peut également s'effectuer par décantation gravitaire.

Selon un mode particulier de l'invention, la solution de lavage récupérée peut être utilisée totalement ou partiellement de nouveau en étape 6), avec ou sans étape de traitement préalable.

Selon un mode particulier, la solution de lavage peut comprendre jusqu'à 55% en poids d'acide 2-acrylamido-2-méthylpropane sulfonique.

Selon un mode particulier de l'invention, la solution de lavage récupérée peut être utilisée totalement ou partiellement dans la solution aqueuse A en étape 4), avec ou sans étape de traitement préalable.

Etape 7

Dans une étape 7) optionnelle, la composition 3 obtenue à l'issue de l'étape 6) est séchée. A titre d'exemples et de manière non limitative, nous pouvons citer l'utilisation de toutes technologies de séchage par convection, conduction ou rayonnement (sécheur à lit fluidisé, lit traversé, séchage sur bande convoyeuse, séchage micro-onde, séchage par rayonnement haute fréquence, infra rouge, séchage par atomisation).

L'opération de séchage peut être réalisée à pression atmosphérique ou bien sous vide.

L'opération de séchage peut être réalisée de manière discontinue (en batch) ou continue.

Pendant le procédé de fabrication, et quelle que soit l'étape, il est possible d'introduire au moins un inhibiteur de polymérisation. Ce dernier peut être choisi de manière non limitative parmi l'hydroquinone, le paraméthoxyphénol, le phénothiazine, le 2,2,6,6-tétraméthylpipéridin-1-yl)oxyl, le 4-hydroxy-2,2,6,6-tétraméthylpipéridin-1-oxyl, les dérivés de phénylène diamines, ou leurs mélanges.

Préférentiellement l'inhibiteur est le paraméthoxyphénol.

La quantité d'inhibiteur introduite par rapport à la quantité de particules comprise dans la composition 1 obtenue à l'issue de l'étape 3) est avantageusement comprise entre 0,001% et 5% en poids, préférentiellement entre 0,01% et 1% en poids.

L'inhibiteur peut être introduit au cours de l'une quelconque des étapes du procédé, comme par exemple par utilisation d'acrylonitrile stabilisé au paraméthoxyphénol. Préférentiellement il est introduit en quantité supplémentaire pendant l'étape 4), plus préférentiellement l'inhibiteur fait partie de la solution aqueuse A introduite en étape 4).

Le procédé peut être effectué de manière continue ou discontinue (en batch).

L'invention et les avantages qui en découlent ressortiront mieux des exemples suivants donnés afin d'illustrer l'invention et non de manière limitative.

## EXEMPLES DE REALISATION DE L'INVENTION

Protocole de préparation de l'acide 2-acrylamido-2-méthylpropane sulfonique

### Exemple 1

Dans un réacteur agité de 2000 ml ayant une double enveloppe, sont ajoutés 1522 grammes d'acrylonitrile contenant 0,4% en poids d'eau et 180 grammes d'acide sulfurique fumant titrant 104% $H_2SO_4$ (18% Oléum). Le mélange sulfonant ainsi obtenu est agité pendant 1 heure et refroidi par la double enveloppe du réacteur qui maintient la température du mélange sulfonant à -20°C.

97 grammes d'isobutylène sont additionnés au mélange sulfonant précédent, à un débit de 1,6 grammes/minute. La température du mélange réactionnel ainsi obtenu est contrôlée à 45°C lors de l'introduction de l'isobutylène. Les particules de l'acide 2-acrylamido-2-méthylpropane sulfonique précipitent dans le mélange réactionnel et le taux de solide est d'environ 20% en poids.

Le mélange réactionnel est filtré sur un filtre de type Büchner, la composition 1 obtenue contient 70% de particules.

100g d'eau, représentant la solution aqueuse A, sont ajoutés dans un réacteur agité de 500ml. La composition 1 récupérée sur le filtre Büchner est ajoutée au réacteur pour obtenir une suspension 1. La température est contrôlée à température ambiante, c'est à dire 25°C.

Après 4 heures de mise en suspension, la suspension 1 obtenue est filtrée sur un filtre de type Büchner. La composition 2 obtenue n'est pas séchée et 148g d'ATBS sont récupérés. Le rendement est de 40% par rapport à l'isobutylène.

322 grammes de liquide contenant de l'eau, de l'acrylonitrile, de l'acide sulfurique et de l'acide 2-acrylamido-2-méthylpropane sulfonique sont récupérés.

### Exemple 2

Les conditions pour l'obtention de la composition 1 sont identiques à l'exemple 1.

100g du liquide obtenu à la fin de l'exemple 1, représentant la solution aqueuse A, sont ajoutés dans un réacteur agité de 500ml. La composition 1 non séchée sur le filtre Büchner est ajoutée au réacteur. La température est contrôlée à température ambiante, c'est à dire 25°C.

Après 3 heures de mise en suspension, la suspension 1 obtenue est filtrée sur un filtre de type Büchner. La composition 2 obtenue est non séchée et 340g d'ATBS sont récupérés. Le rendement est de 91% par rapport à l'isobutylène.

138 grammes de liquide contenant de l'eau, de l'acrylonitrile, de l'acide sulfurique et de l'acide 2-acrylamido-2-méthylpropane sulfonique sont récupérés.

### Exemple 3

Dans un réacteur agité de 2000ml ayant une double enveloppe, sont ajoutés 1215 grammes d'acrylonitrile contenant 0,2% en poids d'eau, 130 grammes d'acide sulfurique (concentration 98%) et 130 grammes d'acide sulfurique fumant titrant 105,62% $H_2SO_4$ (25% Oléum). Le mélange sulfonant ainsi obtenu est agité pendant 1h et refroidi par la double enveloppe du réacteur qui maintient le mélange sulfonant à -20°C.

135 grammes d'isobutylène sont additionnés au mélange sulfonant précédent, à un débit de 1 grammes/minute. La température du mélange réactionnel ainsi obtenu est contrôlée à 40°C lors de l'introduction de l'isobutylène. Les particules de l'acide 2-acrylamido-2-méthylpropane sulfonique précipitent dans le mélange réactionnel et le taux de solide est d'environ 30% en poids.

Le mélange réactionnel est filtré sur un filtre de type Büchner, la composition 1 obtenue contient 70% de cristaux.

120g d'eau, représentant la solution aqueuse A, sont ajoutés dans un réacteur agité de 1000ml. La composition 1 récupérée sur le filtre Büchner est ajoutée au réacteur pour obtenir une suspension 1. La température est contrôlée à température ambiante, c'est à dire 25°C.

Après 3 heures de mise en suspension, la suspension 1 obtenue est filtré sur un filtre de type Büchner. La composition 2 obtenue n'est pas séchée et 250gr d'ATBS sont récupérés. Le rendement est de 50% par rapport à l'isobutylène.

386 grammes de liquide contenant de l'eau, de l'acrylonitrile, de l'acide sulfurique et de l'acide 2-acrylamido-2-méthylpropane sulfonique sont récupérés.

Exemple 4

Dans un réacteur agité de 2000ml ayant une double enveloppe, sont ajoutés 1215 grammes d'acrylonitrile contenant 0,3% en poids d'eau, 100 grammes d'acide sulfurique (concentration 96%) et 130 grammes d'acide sulfurique fumant titrant 105,18% $H_2SO_4$ (23% Oléum). Le mélange sulfonant ainsi obtenu est agité pendant 10mn et refroidi par la double enveloppe du réacteur qui maintient le mélange sulfonant à -20°C.

125 grammes d'isobutylène sont additionnés au mélange sulfonant précédent, à un débit de 1 grammes/minute. La température du mélange réactionnel ainsi obtenu est contrôlée à 40°C lors de l'introduction de l'isobutylène. Les particules de l'acide 2-acrylamido-2-méthylpropane sulfonique précipitent dans le mélange réactionnel et le taux de solide est d'environ 30% en poids.

Le mélange réactionnel est filtré sur un filtre de type Büchner, la composition 1 obtenue contient 70% de cristaux.

110g d'eau, représentant la solution aqueuse A, sont ajoutés dans un réacteur agité de 500ml. La composition 1 récupérée sur le filtre Büchner est ajoutée au réacteur pour obtenir une suspension 1. La température est contrôlée à température ambiante, c'est à dire 25°C.

Après 3 heures de mise en suspension, la suspension 1 obtenue est filtrée sur un filtre de type Büchner. La composition 2 obtenue n'est pas séchée et 235 grammes d'ATBS sont récupérés. Le rendement est de 49% par rapport à l'isobutylène.

354 grammes de liquide contenant de l'eau, de l'acrylonitrile, de l'acide sulfurique et de l'acide 2-acrylamido-2-méthylpropane sulfonique sont récupérés.

## Revendications

1. Procédé de fabrication de l'acide 2-acrylamido-2-méthylpropane sulfonique comprenant au moins les étapes successives suivantes :

   1) mélange d'acrylonitrile avec au moins un composé apporteur de $SO_3$ à une température comprise entre -80 et 30°C pendant au moins 1 seconde afin d'obtenir un mélange sulfonant,
   2) mise en contact et mélange d'isobutylène et du mélange sulfonant avec un ratio molaire $SO_3$:isobutylène compris entre 0,2:1 et 2:1 et un ratio molaire acrylonitrile: isobutylène compris entre 3:1 et 60:1, à une température comprise entre -40 et 100°C pendant un temps compris entre 10 secondes et 300 minutes afin d'obtenir un mélange réactionnel,
   3) séparation solide/liquide du mélange réactionnel et isolement des particules solides contenues dans le mélange réactionnel sous la forme d'une composition 1 dans laquelle les particules solides représentent de 50 à 99% en poids de la composition 1,
   4) mélange de la composition 1 à l'issue de l'étape 3) avec une solution aqueuse A pendant au moins 10 minutes à une température comprise entre -20 et 70°C afin d'obtenir une suspension 1 de cristaux d'acide 2-acrylamido-2-méthylpropane sulfonique,
   5) séparation solide/liquide de la suspension 1 et isolement des cristaux sous la forme d'une composition 2 dans laquelle les cristaux représentent entre 40 et 99 % en poids de la composition 2.

2. Procédé selon la revendication 1, **caractérisé en ce que** le composé apporteur de $SO_3$ est de l'acide sulfurique fumant, utilisé à une concentration comprise entre 100% et 113,5%.

3. Procédé selon l'une quelconque des revendications précédentes, **caractérisé en ce que** l'étape 1) comprend le mélange d'acrylonitrile avec un composé apporteur de $SO_3$ dans un solvant 1, choisi avantageusement dans le groupe comprenant l'anhydride acétique, les acides carboxyliques tel que l'acide acétique, les nitriles, les alcools, les amines, les alcanes, les amides, les éthers, les aromatiques, les acides alkylsulfoniques et la phase liquide résultant de la séparation liquide/solide de l'étape 3).

4. Procédé selon la revendication 3, **caractérisé en ce que** le solvant 1 est l'acrylonitrile.

5. Procédé selon l'une quelconque des revendications précédentes, **caractérisé en ce que** l'isobutylène est introduit, lors de l'étape 2), dissous dans un solvant 2, préférentiellement de l'acrylonitrile ou la phase liquide résultant de la séparation liquide/solide de l'étape 3).

6. Procédé selon l'une quelconque des revendications précédentes, **caractérisé en ce que** le ratio molaire $SO_3$:isobutylène de l'étape 2) est compris entre 0,4:1 et 1,5:1, préférentiellement entre 0,7:1 et 1,2:1.

7. Procédé selon l'une quelconque des revendications 3 à 6, **caractérisé en ce que** le mélange réactionnel est concentré en particules d'acide 2-acrylamido-2-méthylpropane sulfonique par évaporation des solvants 1 et 2 avant l'étape de séparation solide/liquide.

8. Procédé selon l'une quelconque des revendications précédentes, **caractérisé en ce que** le ratio en poids de solution aqueuse A mélangée, lors de l'étape 4), avec la composition 1 de l'étape 3) est compris entre 0,05:1 et 1:1, plus préférentiellement entre 0,15:1 et 0,9:1.

9. Procédé selon l'une quelconque des revendications précédentes, **caractérisé en ce que** la composition 1 est lavée avec un solvant 3, de préférence une solution d'acrylonitrile.

10. Procédé selon l'une quelconque des revendications précédentes, **caractérisé en ce que** la solution aqueuse A de l'étape 4) comprend jusqu'à 20% d'un solvant 4 organique, préférentiellement de 0 à 15 % de solvant 4 organique, plus préférentiellement de 2 à 10% de solvant 4 organique.

11. Procédé selon la revendication 9, **caractérisé en ce que** le solvant 4 organique est l'acrylonitrile.

12. Procédé selon l'une des revendications 1 à 8, **caractérisé en ce que** la solution aqueuse A de l'étape 4) comprend au moins 80% en poids d'eau et jusqu'à 20% en poids d'acide inorganique, préférentiellement entre 80% et 99% en poids d'eau et de 1% à 20% en poids d'acide inorganique, plus préférentiellement entre 85% et 98% en poids d'eau et 2% à 15% en poids d'acide inorganique.

13. Procédé selon la revendication 11, **caractérisé en ce que** l'acide inorganique est l'acide sulfurique.

14. Procédé selon l'une des revendications précédentes, **caractérisé en ce que** la phase liquide obtenue à l'issue de l'étape 5) de séparation solide/liquide de la suspension 1 sert totalement ou partiellement de solution aqueuse A dans l'étape 4).

15. Procédé selon l'une des revendications précédentes, **caractérisé en ce que** la composition 2 de l'étape 5) est lavée à l'aide d'une solution de lavage, avantageusement une solution aqueuse comprenant jusqu'à 20% de solvant 4 organique.

## Patentansprüche

1. Verfahren zur Herstellung von 2- Acrylamid- 2-methylpropansulfonsäure, das mindestens die nachstehenden aufeinanderfolgenden Schritte beinhaltet:

   1) Mischen von Acrylnitril mit mindestens einer $S0_3$- erhöhenden Verbindung bei einer Temperatur zwischen -80 und 30° C, mindestens eine Sekunde lang, um eine Sulfonierungsmischung zu erhalten,
   2) in Kontakt bringen und Mischung von Isobutylen und der Sulfonierungsmischung mit einem Molverhältnis $S0_3$:Isobutylen zwischen 0,2:1 und 2:1 und einem Molverhältnis Acrylnitril:Isobutylen zwischen 3:1 und 60:1, bei einer Temperatur zwischen -40 und 100°C während einer Zeitspanne zwischen 10 Sekunden und 300 Minuten, um ein Reaktionsgemisch zu erhalten,
   3) Trennung Feststoff/ Flüssigkeit des Reaktionsgemischs und Isolierung der im Reaktionsgemisch enthaltenen festen Teilchen, in Form einer Zusammensetzung 1, in der die festen Teilchen zwischen 50 und 99 Gewichts-% der Zusammensetzung bilden.
   4) Mischen der Zusammensetzung 1 am Ende von Schritt 3) mit einer wässrigen Lösung A, mindestens 10 Minuten lang, bei einer Temperatur zwischen -20 und $70^0$ C um eine Suspension 1 von Kristallen der 2-Acrylamid-2-methylpropansulfonsäure zu erhalten.
   5) Trennung Feststoff/ Flüssigkeit der Suspension 1 und Isolierung der Kristalle in Form einer Zusammensetzung 2, in der die Kristalle zwischen 40 und 99 Gewichts-% der Zusammensetzung 2 bilden.

2. Verfahren nach Anspruch 1, **dadurch gekennzeichnet, dass** es sich bei der $S0_3$-erhöhenden Verbindung um rauchende Schwefelsäure handelt, die mit einer Konzentration zwischen 100% und 113,5 % eingesetzt wird.

3. Verfahren nach irgendeinem der vorangehenden Ansprüche, **dadurch gekennzeichnet, dass** Schritt 1) das Mischen von Acrylnitril mit einer $S0_3$-erhöhenden Verbindung in einem Lösungsmittel 1 umfasst, das vorteilhafterweise

ausgewählt wird aus der Gruppe mit Essigsäureanhydrid, den Carboxylsäuren, wie Essigsäure, Nitrilen, Alkoholen, Aminen, Alkanen, Amiden, Ether, Aromaten, Alkylsulfon- Säuren und die flüssige Phase, die sich aus der Trennung Flüssigkeit/ Feststoffe des Schritts 3) ergibt.

**4.** Verfahren gemäß Anspruch 3, **dadurch gekennzeichnet, dass** es sich bei dem Lösungsmittel um Acrylnitril handelt.

**5.** Verfahren nach irgendeinem der vorangehenden Ansprüche **dadurch gekennzeichnet, dass** das Isobutylen in Schritt 2) hinzugefügt wird, aufgelöst in einem Lösungsmittel 2, vorzugsweise Acrylnitril oder der flüssigen Phase, die sich aus der Trennung Flüssigkeit/ Feststoffe des Schritts 3) ergibt.

**6.** Verfahren nach irgendeinem der vorangehenden Ansprüche, **dadurch gekennzeichnet, dass** das Molverhältnis $S0_3$:Isobutylen aus Schritt 2) zwischen 0,4:1 und 1,5:1 liegt, am besten zwischen 0,7:1 und 1,2:1.

**7.** Verfahren nach einem beliebigen der Ansprüche 3 bis 6, **dadurch gekennzeichnet, dass** das Reaktionsgemisch eine hohe Konzentration von 2-Acrylamid- 2-methylpropansulfonsäure- Teilchen aufweist, durch Verdampfen der Lösungsmittel 1 und 2 vor dem Schritt der Trennung Flüssigkeit/ Feststoffe.

**8.** Verfahren nach irgendeinem der vorangehenden Ansprüche, **dadurch gekennzeichnet, dass** das Gewichtsverhältnis der wässrigen Lösung A vermischt während Schritt 4), mit der Zusammensetzung 1 des Schritts 3) zwischen 0,05:1 und 1:1 liegt, noch besser zwischen 0,15:1 und 0,9:1.

**9.** Verfahren nach irgendeinem der vorangehenden Ansprüche, **dadurch gekennzeichnet, dass** die Zusammensetzung 1 mit einem Lösungsmittel 3 gewaschen wird, am besten einer Acrylnitril- Lösung.

**10.** Verfahren nach irgendeinem der vorangehenden Ansprüche, **dadurch gekennzeichnet, dass** die wässrige Lösung A aus Schritt 4) bis zu 20% eines organischen Lösungsmittels 4 enthält, vorzugsweise zwischen 0 und 15 % organisches Lösungsmittel 4, noch besser zwischen 2 und 10 % organisches Lösungsmittel 4.

**11.** Verfahren gemäß Anspruch 9, **dadurch gekennzeichnet, dass** es sich bei dem organischen Lösungsmittel 4 um Acrylnitril handelt.

**12.** Verfahren nach irgendeinem der Ansprüche 1 bis 8, **dadurch gekennzeichnet, dass** die wässrige Lösung A aus Schritt 4) mindestens 80 Gewichts-% Wasser und bis zu 20 Gewichts-% einer anorganischen Säure enthält, vorzugsweise zwischen 80% und 99 Gewichts-% Wasser und 1 % bis 20% Gewichts-% anorganische Säure, noch besser zwischen 85% und 98 Gewichts-% Wasser und 2% bis15 Gewichts-% anorganische Säure.

**13.** Verfahren nach Anspruch 1, **dadurch gekennzeichnet, dass** es sich bei der anorganischen Säure um Schwefelsäure handelt.

**14.** Verfahren nach irgendeinem der vorangehenden Ansprüche, **dadurch gekennzeichnet, dass** die flüssige Phase der Suspension 1, die sich aus der Trennung Flüssigkeit/ Feststoffe des Schritts 5) ergibt, ganz oder teilweise als wässrige Lösung A in Schritt 4) dient.

**15.** Verfahren nach irgendeinem der vorangehenden Ansprüche, **dadurch gekennzeichnet, dass** die Verbindung 2 des Schritts 5) mit einer Waschlösung gewaschen wird, vorteilhafterweise eine wässrige Lösung, die bis zu 20% des organischen Lösungsmittels 4 enthält.

## Claims

**1.** A production process for 2-acrylamido-2-methylpropane sulfonic acid including at least the following successive steps:

1) mixing of acrylonitrile with at least one compound contributing $SO_3$ at a temperature included between -80 and 30 °C for at least one second in order to obtain a sulfonating mixture;
2) placing in contact and mixing isobutylene and the sulfonating mixture with a molar ratio of $SO_3$ to isobutylene included between 0.2:1 and 2:1 and a molar ratio of acrylonitrile to isobutylene included between 3:1 and 60:1 at a temperature included between -40 and 100 °C for a time included between 10 seconds and 300 minutes

in order to obtain a reaction mixture;

3) solid/liquid separation of the reaction mixture and isolation of the solid particles contained in the reaction mixture in the form of a composition 1 in which the solid particles represent 50 to 99 % by weight of the composition 1;

4) mixing composition 1 at the end of step 3) with an aqueous solution A for at least 10 minutes at a temperature included between -20 and 70 °C in order to obtain a suspension 1 of 2-acrylamido-2-methylpropane sulfonic acid crystals;

5) solid/liquid separation of the suspension 1 and isolation of the crystals in the form of a composition 2 in which the crystals represent between 40 and 99 % by weight of the composition 2.

2. The process according to claim 1, **characterized in that** $SO_3$ contributing compound is fuming sulfuric acid, used at a concentration included between 100 % and 113.5 %.

3. The process according to any one of the preceding claims, **characterized in that** step 1) includes mixing acrylonitrile with at least one compound contributing $SO_3$ in a solvent 1, advantageously chosen from the group including acetic anhydride, carboxylic acids such as acetic acid, nitriles, alcohols, amines, alkanes, amides, ethers, aromatics, alkylsulfonic acids and the liquid phase resulting from the liquid/solid separation from step 3).

4. The process according to claim 3, **characterized in that** the solvent 1 is acrylonitrile.

5. The process according to any one of the preceding claims, **characterized in that** isobutylene is added, during step 2), dissolved in a solvent 2, preferably acrylonitrile or the liquid phase resulting from the liquid/solid separation from step 3.

6. The process according to any one of the preceding claims, **characterized in that** the molar ratio of $SO_3$ to isobutylene from step 2) is included between 0.4:1 and 1.5:1 and preferably between 0.7:1 and 1.2:1.

7. The process according to any one of claims 3 to 6, **characterized in that** 2-acrylamido-2-methylpropane sulfonic acid particles are concentrated in the reaction mixture by evaporation of solvents 1 and 2 before the solid/liquid separation step.

8. The process according to any one of the preceding claims, **characterized in that** the ratio by weight of aqueous solution A mixed, during step 4), with the composition 1 from step 3 is included between 0.05:1 and 1:1, and more preferably between 0.15:1 and 0.9:1.

9. The process according to any one of the preceding claims, **characterized in that** composition 1 is washed with a solvent 3, preferably an acrylonitrile solution.

10. The process according to any one of the preceding claims, **characterized in that** the aqueous solution A of step 4 includes up to 20 % of organic solvent 4, preferably from 0 to 15 % of organic solvent 4, more preferably from 2 to 10 % of organic solvent 4.

11. The process according to claim 9, **characterized in that** the organic solvent 4 is acrylonitrile.

12. The process according to any one of claims 1 to 8 **characterized in that** the aqueous solution A from step 4) includes at least 80 % by weight of water and up to 20 % by weight of inorganic acid, preferably between 80 % and 99 % by weight of water and from 1 % to 20 % by weight of inorganic acid, more preferably between 85 % and 98 % by weight of water and 2 % to 15 % by weight of inorganic acid.

13. The process according to claim 11, **characterized in that** the inorganic acid is sulfuric acid.

14. The process according to any one of the preceding claims, **characterized in that** the liquid phase resulting at the end of the solid/liquid separation of the suspension 1 in step 5) serves wholly or partially as aqueous solution A in step 4).

15. The process according to any one of the preceding claims, **characterized in that** the composition 2 of step 5) is washed using a washing solution, advantageously an aqueous solution including up to 20 % by weight of organic solvent 4.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- US 6448347 B
- CN 102351744
- WO 2009072480 A
- US 4337215 A
- CN 103664709


### Littérature non-brevet citée dans la description


- On the Ritter synthesis of N-tert-butylacrylamide, reaction between tert-butylalcohol and acrylonitrile in non-aqueous system. *Iranian J. of Polymer Science and Technology,* 1995, vol. 4 (1), 42-49